# EUROPÄISCHE PATENTSCHRIFT

(11) **EP 2 620 520 B1**
(45) Veröffentlichungstag und Bekanntmachung des Hinweises auf die Patenterteilung: **20.03.2024**
(21) Anmeldenummer: 12191203.4
(22) Anmeldetag: 05.11.2012
(51) Int. Cl.: C23C 16/52, C23C 16/04, G01N 33/00

(54) **Qualitätskontrolle von Behälterbeschichtungen**
Quality control of container coatings
Contrôle de qualité de revêtements de récipients

(30) Priorität: 24.01.2012 DE 102012200976
(43) Veröffentlichungstag der Anmeldung: 31.07.2013
(73) Patentinhaber: Krones AG, 93073 Neutraubling (DE)
(72) Erfinder: Krüger, Jochen, 93073 Neutraubling (DE)
(74) Vertreter: Grünecker Patent- und Rechtsanwälte PartG mbB

(56) Entgegenhaltungen:
- EP-A1- 0 647 847
- WO-A1-2011/143509
- DE-A1- 4 305 799
- DE-A1- 10 354 625
- US-A1- 2006 169 026

## Beschreibung

### Stand der Technik

Die Erfindung betrifft ein Verfahren und eine Vorrichtung zur Qualitätskontrolle von Beschichtungen von Behältnissen, insbesondere der Kontrolle von durch Plasmabehandlung erzeugten Beschichtungen von Kunststoffflaschen.

Bei der Plasmaabscheidung von Gas-Barriereschichten in Kunststoffflaschen werden Schichten, beispielsweise aus amorphem Siliziumoxid (SiOx) oder Kohlenstoffverbindungen (z.B. Diamond Like Carbon "DLC") abgeschieden, mit dem Ziel, die Permeation von Gasen wie CO2 oder Sauerstoff durch das Kunststoff-Material hindurch zu verhindern. Bei der Innenbeschichtung von Flaschen, beispielsweise Kunststoffflaschen aus Polyethylenterephthalat (PET), kann zusätzlich die Migration von Fremdstoffen aus dem Kunststoff in das Produkt verhindert werden. Als Beispiel für solche Fremdstoffe seien hier für PET vor allem das Acetaldehyd oder Antimon genannt; aber auch Flaschenmaterialen, die auf anderen Kunststoffen basieren enthalten z.B. Weichmacher, die teilweise nur schwach an das Trägermaterial gebunden sind.

Insbesondere nach einer thermischen Behandlung der Flaschen, wie einer Plasmabehandlung, wird in den Flaschen das Auslösen von unerwünschten Stoffen deutlich begünstigt. Die Erfahrung hat beispielsweise gezeigt, dass PET-Flaschen bereits nach einer thermischen Behandlung, bzw. nach einem Plasmabeschichtungsprozess mit einer mangelhaften Schichtqualität unangenehm stark nach Acetaldehyd riechen.

Darüber hinaus ist es Zweck einer Plasmabeschichtung die Aufnahme von Sterilisationsmedien wie PAA oder H2O2 in das Flaschenmaterial zu verhindern, da dies zu einer verzögerten Abgabe von unerwünschten Fremdstoffen an das sich in der Flasche befindenden Produkt führen kann.

Die Dicken dieser Plasmaschichten, im Folgenden auch Barriereschichten genannt, liegen dabei im Bereich von zehn bis einige hundert Nanometer und sind insbesondere im Fall von Siliziumoxid unsichtbar.

Zur Überwachung der Beschichtungsqualität sind dabei folgende Methoden bekannt:
Für die Erfassung von Permeationsstoffen wie von Gasen, die durch die Flaschenwand dringen, können klassische Messvorrichtungen, z.B. von Firmen wie PreSens oder Mocon (Ox-tran) verwendet werden.

Die Beschichtung kann auch mit modernen Methoden der Schichtanalyse untersucht werden, z.B. mit Fouriertransformations-Infrarotspektroskopie (FTIR) Techniken wie in der US6531193 oder der EP1273677 beschrieben oder durch Ellipsometrie (siehe z.B. J. Electrochem. Soc., Volume 138, Issue 11, pp. 3266-3275 , 1991).

Eine weitere Methode ist ein sogenannter Säuretest, bei dem die Flaschen einem aggressiven Medium, wie z.B. konzentrierter Schwefelsäure ausgesetzt werden. Unbeschichtete Flaschen werden durch das Mittel angegriffen und trüb, beschichtete Flaschen weisen einen gewissen Schutz gegenüber dem Medium auf.

Alle diese Methoden haben den Nachteil, dass sie sehr lange dauern (einige Minuten bis einige Tage), oder dass die Flasche zur Analyse zerstört werden muss.

Darüber hinaus ist bekannt, wie beispielsweise in der US020080292781A und der DE102010012501 beschrieben, dass eine schnellere Kontrolle der Plasmabeschichtung mittels lichtspektroskopischer Untersuchungsverfahren erfolgen kann. Dabei wird die Intensität bestimmter Spektrallinien des sich in der Flasche befindenden Plasmas mit einer vordefinierten Referenzintensität verglichen, und bei hinreichender Übereinstimmung der Spektrallinienintensitäten davon ausgegangen, dass die Plasmabeschichtung funktioniert hat. Diese Methode prüft jedoch nur indirekt die Plasmabeschichtung, und eine fehlerfreie komplette Plasmabeschichtung, die eine intakte Gasbarriere darstellt, kann nicht mit Bestimmtheit festgestellt werden.

Weiterhin sind spurengasanalytische Messmethoden aus der DE10354625 bekannt, die Stichproben plasmabeschichteter Behälter untersucht, wobei unerwünschte aus dem Behältermaterial austretende Fremdstoffe bestimmt werden.

WO2011143509 diskutiert massenspektrometrische Bestimmungen der Ausgasungen von Plasmabeschichtungen von Behältern, wobei die Gase inline, d.h. in der Geschwindigkeit in der sie den Produktions- bzw. Behandlungsprozess durchlaufen, von einer Messeinrichtung untersucht werden.

US2006169026 offenbart massenspektrometrische Untersuchungen plasmabeschichteter Behälter, wobei die Durchlässigkeit von Gasen, beispielsweise Wasserstoff, Sauerstoff oder Argon, durch die Beschichtung bestimmt wird. Unerwünschte Fremdstoffe in dem Behältermaterial (Feuchtigkeit und organische Verbindungen) werden vor den Messungen entfernt.

### Aufgabe

Es ist somit Aufgabe der Erfindung eine Vorrichtung und ein Verfahren anzugeben, um die Kontrolle von beschichteten Behältnissen, beispielsweise von Kunststoffflaschen, die durch Plasmabehandlung beschichtet wurden, zu verbessern.

### Lösung

Dies wird erfindungsgemäß durch ein Verfahren nach Anspruch 1 und eine Vorrichtung nach Anspruch 8 erreicht. Vorteilhafte Ausführungsformen und Weiterbildungen sind Gegenstand der Unteransprüche.

Die Erfindung macht sich zu Nutze, dass bei Behältnissen, wie Kunststoffflaschen, welche mit einer durch Plasmabeschichtung erzeugten Barriereschicht versehen sind, das Austreten unerwünschter Fremdstoffe aus dem Behältermaterial nahezu vollständig unterdrückt wird. Behälter mit einer kompletten und intakten Barriereschicht riechen dem zufolge neutral. In einem Verfahren nach Anspruch 1 kann mittels spurengasanalytischer Untersuchung, z.B. mit einem Massenspektrometer, ein durch eine Plasmabehandlung beschichteter Behälter auf aus dem Behältermaterial austretende unerwünschte Fremdstoffe, wie beispielsweise Acetaldehyd und/oder Antimon, untersucht werden.

Eine spurengasanalytische Untersuchung hat den Vorteil, dass innerhalb kurzer Zeit, beispielsweise weniger als 0.1, 1, 10, 100 ms pro Behälter, eine Prüfung der Güte, bzw. Unversehrtheit der durch Plasmabehandlung erzeugten Behältnisbeschichtung erzielt werden kann, in dem die Anwesenheit oder Abwesenheit von aus dem Behältermaterial austretende unerwünschte Fremdstoffen festgestellt werden kann.

Genannte kurze Untersuchungszeiten reichen dafür aus, dass die Behälter direkt ("inline") auf austretende unerwünschte Fremdstoffe untersucht werden können, d.h. in der Geschwindigkeit, in der sie den Produktions-bzw. Behandlungsprozess durchlaufen. Das hat den Vorteil, dass die Behälter nach der Beschichtung nicht umständlich stichprobenartig und zeitaufwändig gemessen werden müssen und jeder einzelne Behälter gemessen werden kann.

Es ist dabei möglich, dass man direkt nach einer Beschichtungsbehandlung, beispielsweise mittels Plasmabehandlung, und/oder nach einer anderen thermischen Behandlung des Behälters, die sich im Behälter befindende Luft, spurengasanalytisch auf aus dem Behältermaterial austretende unerwünschte Fremdstoffen untersucht, ohne dass zuvor der Behälter mit einem Normgas, wie beispielsweise technisch gereinigter Luft, gespült werden muss. Dazu kann beispielsweise ein Schnüffler verwendet werden, der in den Behälter eingeführt werden kann, oder sich unmittelbar (innerhalb eines Abstand von 0,1, 1, 5, oder 10 cm, bevorzugt 1 bis 2 cm, von der Behälteröffnung) über der Behälteröffnung befinden kann, und der einen Teil der sich im Behälter befindlichen Luft an eine Messeinrichtung, beispielsweise einen Spurengasanalysator, weiterleiten, um diese auf die Anwesenheit, bzw. Konzentration oder Abwesenheit von aus dem Behältermaterial austretende unerwünschte Fremdstoffen untersuchen zu können.

Vorteilhafterweise kann aber auch ein Normgas, wie beispielsweise Umgebungsluft, technisch gereinigte Luft, ein reaktionsträges Inertgas, ein Edelgas, ein Edelgasgemisch oder eine Kombination genannter Gase, in das zu untersuchende Behältnis eingeblasen/gespült werden, und die aus dem Behältnis wieder austretende Mischung aus Normgas und etwaigen aus dem Behältermaterial austretenden Stoffen, auch Probegas genannt, von einer Messeinrichtung auf aus dem Behältermaterial austretende unerwünschte Fremdstoffen untersucht werden.

Die Güte der mittels Plasmabehandlung erzeugten Barriereschicht kann beispielsweise dadurch quantifiziert werden, dass ein Grenzwert für die Konzentration eines unerwünschten Fremdstoffes festgelegt werden kann, und beispielsweise bei Überschreitungen des Konzentrationsgrenzwertes von mehr als 0.1, 1, 5, 10 oder 100 %, der untersuchte Behälter als beispielsweise unzureichend beschichtet eingestuft und/oder markiert werden kann, und/oder aus der Produktion endgültig ausgeleitet wird und/oder einer erneuten Plasmabeschichtungsbehandlung zugeführt wird. Die Festlegung der Grenzwerte für die Konzentration eines unerwünschten Fremdstoffes kann dabei zusätzlich von der Temperatur des zu untersuchenden Behälters, beispielsweise der Innentemperatur des Behälters, abhängen. So ist beispielsweise denkbar, dass bei steigender Temperatur, z.B. bei Temperaturen von 30, 50 °C oder mehr des zu untersuchenden Behälters, ein höherer Grenzwert für die Konzentration eines unerwünschten Fremdstoffes toleriert werden kann, als beispielsweise im Vergleich zu Behältern mit Innentemperaturen die bei Zimmertemperatur liegen. Durch eine solche mögliche von der Innentemperatur des Behälters abhängige Grenzwertfestlegung für die Konzentration eines unerwünschten Fremdstoffes, kann die Untersuchung auf unerwünschte Fremdstoffe die Innentemperaturschwankungen zu untersuchender Behälter berücksichtigen. Innentemperaturschwankungen zu untersuchender Behälter können dabei beispielsweise bedingt durch Schwankungen in den Umgebungsbedingungen, wie z.B. Sommer/Winter, Tag/Nacht, Zugluft, etc. auftreten.

In einer weiteren vorteilhaften Ausführung der Erfindung ist es möglich, dass das zu untersuchende Behältnis vor der Plasmabeschichtungsbehandlung mit einem Tracerstoff / mit Tracerstoffen, beispielsweise mit einem lebensmitteltechnisch unkritischen Stoff wie Xenon, geimpft wird, und das Behältnis dann nach der Plasmabeschichtungsbehandlung spurengasanalytisch auf aus den Behältermaterial austretende Tracerstoffe untersucht wird. Hierzu kann beispielsweise ein Tracer der Blasluft einer Streckblasmaschine zugegeben werden, um ein Behältnis bei dessen Formung zu impfen.

Die Figuren stellen beispielhaft dar:
**Fig. 1****:** Ein beispielhaftes Verfahrensschema.
**Fig. 2****:** Eine beispielhafte Vorrichtungsanordnung.

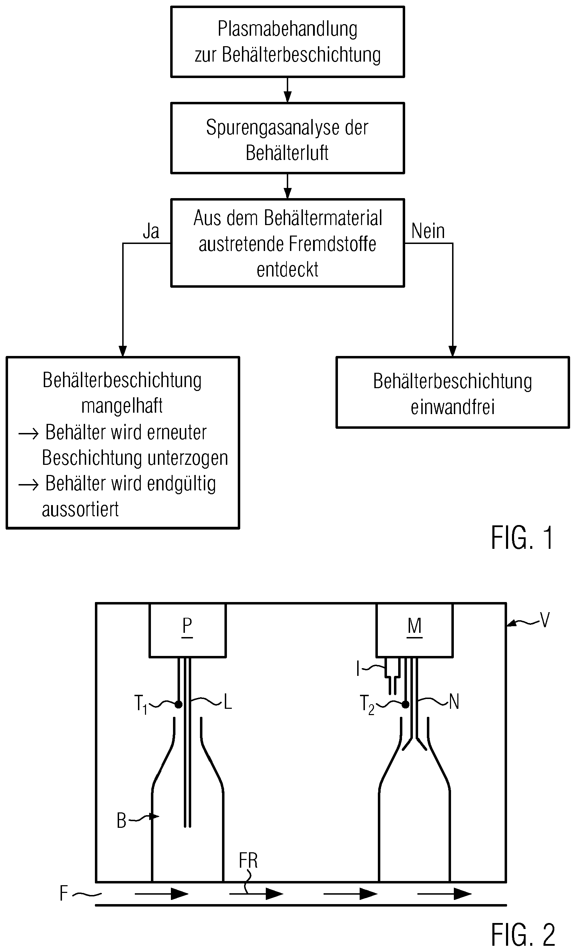

Die **Fig. 1** beschreibt beispielhaft einige technische Aspekte zum besseren Verständnis der beanspruchten Verfahrensausführung.

Dabei kann zunächst ein Behälter beschichtet werden, beispielsweise durch eine Plasmabehandlung. Anschließend kann die Beschichtung mittels einer Spurengasanalyse überprüft werden, in dem beispielsweise die Behälterluft oder Behälterprobegas auf aus dem Behältermaterial austretende unerwünschte Fremdstoffe oder aus dem Behältermaterial austretende Tracer untersucht wird. Werden keine unerwünschten Fremdstoffe und/oder Tracer, die aus dem Behältermaterial austreten, bzw. Konzentrationen besagter Stoffe unterhalb vordefinierter Grenzwerte, detektiert, kann die Beschichtung beispielsweise als einwandfrei eingestuft werden.

Im Fall, dass jedoch unerwünschte aus dem Behältermaterial austretende Fremdstoffe und/oder Tracer bzw. Konzentrationen besagter Stoffe, die oberhalb vordefinierter Grenzwerte liegen, gemessen werden, kann die Behälterbeschichtung beispielsweise als mangelhaft und/oder ungenügend eingestuft werden, und der beanstandete Behälter kann entweder einer neuen Beschichtungsbehandlung zugeführt werden oder endgültig aus dem Produktionsprozess ausgeleitet und entfernt werden.

Die **Fig. 2** zeigt schematisch ein Beispiel für eine vorteilhafte Ausführung einer Vorrichtung V zur Beschichtung von Behältern, beispielsweise mittels Plasmabehandlung, und für anschließende Untersuchung beschichteter Behälter auf aus dem Behältermaterial austretende unerwünschte Fremdstoffe oder aus dem Behältermaterial austretende Tracer. Die Vorrichtung kann dabei eine Einheit zur Beschichtung von Behältnissen, beispielsweise eine Plasmabehandlungseinheit P aufweisen, welche beispielsweise über eine Gaslanze L dem Behälter Gas zuführen kann und ein Plasma zur Behalterbeschichtung zünden kann. Die Vorrichtung V kann über eine Behälterbeförderungseinrichtung F verfügen, die den beschichteten Behälter B zu einer Messeinrichtung M führen kann, wo der Behälter B, beispielsweise durch Spurengasanalyse, auf aus dem Behältermaterial austretende unerwünschte Fremdstoffe oder Tracer untersucht werden kann. Dabei kann die Messeinrichtung M beispielsweise über einen Schnüffler N verfügen, der die sich im Behälter B befindliche Luft teilweise an die Messeinrichtung M weiterleiten kann.

Darüber hinaus ist es ebenso möglich, dass die Messeinrichtung M zusätzlich wenigstens eine Einblaseinheit I und wenigstens einen Schnüffler N aufweist, und die wenigstens eine Einblaseinheit I ein Normgas in einen zu untersuchenden Behälter B einblasen kann, und der wenigstens eine Schnüffler N mindestens einen Teil des aus einem zu untersuchenden Behältnis austretenden Probegases K aufnehmen und an die Messeinrichtung M weiterleiten kann.

Zur Kontrolle der Temperatur der zu behandelnden und zu untersuchenden Behälter kann die Vorrichtung V zur Beschichtung der Behälter B und anschließender Kontrolle der Beschichtung über wenigstens einen Temperatursensor verfügen, der beispielsweise die Innentemperatur des zu behandelnden und/oder zu untersuchenden Behälters B messen kann. In Fig. 2 sind beispielhaft zwei Temperatursensoren T1 und T2 dargestellt, welche in einen Behälter eingeführt werden können, um z.B. seine Innentemperatur zu messen. So kann z.B. die Plasmabehandlungseinheit P einen Temperatursensor T1 aufweisen und/oder Messeinrichtung M einen Temperatursensor T2.

Es folgt 1 Blatt mit 2 Figuren, wobei die Bezugszeichen wie folgt belegt sind:
- **B**: Behälter, beispielsweise eine Kunststoffflasche.
- **V**: Vorrichtung zur Beschichtung von Behältern, beispielsweise mittels Plasmabehandlung, und für anschließende Untersuchung beschichteter Behälter auf aus dem Behältermaterial austretende unerwünschte Fremdstoffe oder aus dem Behältermaterial austretende Tracer.
- **P**: Plasmabehandlungseinheit zur Beschichtung von Behältern.
- **L**: Gaslanze für Gaszufuhr und Zündung des Plasmas.
- **M**: Messeinrichtung, beispielsweise ein Massenspektrometer, zur Spurengasanalyse der Behälterluft, bzw. des Probegases, auf aus dem Behältermaterial austretende unerwünschte Fremdstoffe oder aus dem Behältermaterial austretende Tracer.
- **N**: Schnüffler, der Behälterluft und/oder Probegas der Messeinrichtung zuführen kann.
- **F**: Beförderungseinheit für Behälter.
- **FR**: Bewegungsrichtung der Beförderungseinheit für Behälter.
- **T1, T2**: Temperatursensor(en).

## Patentansprüche

1. Verfahren zur Untersuchung und Kontrolle von mittels Plasmabehandlung beschichteten Behältern, wie beispielsweise Kunststoffflaschen die z.B. mit amorphem Siliziumoxid oder Kohlenstoffverbindungen beschichtet sind, welches beinhaltet, dass die Behälter nach einer Plasmabeschichtungsbehandlung, spurengasanalytisch, beispielsweise massenspektrometrisch, von einer Messeinrichtung inline, d.h. in der Geschwindigkeit in der sie den Produktions- bzw. Behandlungsprozess durchlaufen, auf aus dem Behältermaterial austretende unerwünschte Fremdstoffe, wie beispielsweise Acetaldehyd und/oder Antimon, untersucht werden.

2. Verfahren nach Anspruch 1, welches beinhaltet, dass die Behälter inline innerhalb einer Untersuchungszeit pro Behälter von weniger als 0.1, 1, 10, oder 100 ms auf unerwünschte Fremdstoffe untersucht werden.

3. Verfahren nach einem der vorigen Ansprüche, welches beinhaltet, dass mittels eines Schnüfflers, die sich im Behältnis befindliche Luft auf aus dem Behältermaterial austretende unerwünschte Fremdstoffe untersucht wird.

4. Verfahren nach einem der vorigen Ansprüche, welches beinhaltet, dass ein Normgas, wie beispielsweise Umgebungsluft, technisch gereinigte Luft, ein reaktionsträges Inertgas, ein Edelgas, ein Edelgasgemisch oder einer Kombination genannter Gase, in einen zu untersuchenden Behälter eingeblasen wird und mindestens ein Teil des aus dem Behälter austretenden Probegases von einer Messeinrichtung auf aus dem Behältermaterial austretende unerwünschte Fremdstoffe untersucht wird.

5. Verfahren nach einem der vorigen Ansprüche, welches beinhaltet, dass bei Feststellung des Überschreitens eines vordefinierten Grenzwertes in der Konzentration, beispielsweise bei Überschreitungen des Grenzwertes von mehr als 0.1, 1, 5, 10 oder 100 %, bezüglich eines bestimmten unerwünschten Fremdstoffes, der untersuchte Behälter beispielsweise als unzureichend beschichtet eingestuft, und/oder markiert werden kann, und/oder aus der Produktion endgültig ausgeleitet wird, und/oder einer erneuten Plasmabeschichtungsbehandlung zugeführt wird.

6. Verfahren nach Anspruch 5, welches beinhaltet, dass der vordefinierte Grenzwert in der Konzentration bezüglich eines bestimmten unerwünschten Fremdstoffes abhängig ist von der Temperatur des zu untersuchenden Behälters, beispielsweise der Innentemperatur des zu untersuchenden Behälters, wobei beispielsweise die tolerierbaren vordefinierte Grenzwert in der Konzentration bezüglich eines bestimmten unerwünschten Fremdstoffes für einen Behälter mit einer Innentemperatur von 30, 50 °C oder mehr, über den vordefinierten Grenzwerten für einen Behälter mit einer Innentemperatur, die gleich der Zimmertemperatur ist, liegen.

7. Verfahren nach einem der vorigen Ansprüche, welches beinhaltet, dass das zu untersuchende Behältnis vor der Plasmabeschichtungsbehandlung mit einem Tracerstoff / mit Tracerstoffen, beispielsweise mit einem Lebensmitteltechnisch unkritischen Stoff wie Xenon, geimpft wird, und das Behältnis nach der Plasmabeschichtungsbehandlung spurengasanalytisch auf aus den Behältermaterial austretende Tracerstoffe untersucht wird.

8. Vorrichtung zur Beschichtung von Behältnissen, beispielsweise Kunststoffflaschen, mittels Plasmabehandlung, sowie zur Untersuchung und Kontrolle der durch die Plasmabehandlung erfolgten Behältnisbeschichtungen, mit wenigstens einer Plasmabehandlungseinheit zur Beschichtung von Behältnissen, und mit wenigstens einer als Massenspektrometer ausgeführten Messeinrichtung, **dadurch gekennzeichnet, dass** die Messeinrichtung (M) so konfiguriert ist, dass sie die von der Plasmabehandlungseinheit (P) beschichteten Behältnisse auf aus dem Behältermaterial austretende unerwünschte Fremdstoffe inline, d.h. in der Geschwindigkeit in der die Behältnisse den Produktions- bzw. Behandlungsprozess durchlaufen, untersuchen kann.

9. Vorrichtung nach Anspruch 8, **dadurch gekennzeichnet, dass** die Messeinrichtung zusätzlich wenigstens einen Schnüffler aufweist, so konfiguriert, dass der wenigstens eine Schnüffler (N) mindestens einen Teil der sich im zu untersuchenden Behältnis befindlichen Luft aufnehmen und an die Messeinrichtung (M) weiterleiten kann.

10. Vorrichtung nach einem der vorigen Ansprüche 8 bis 9, **dadurch gekennzeichnet, dass** die Messeinrichtung zusätzlich wenigstens eine Einblaseinheit und wenigstens einen Schnüffler aufweist, so konfiguriert, dass die wenigstens eine Einblaseinheit (I) ein Normgas in einen zu untersuchenden Behälter (B) einblasen kann, und der wenigstens eine Schnüffler (N) mindestens einen Teil des aus einem zu untersuchenden Behältnis austretenden Probegases (K) aufnehmen und an die Messeinrichtung (M) weiterleiten kann.

11. Vorrichtung nach einem der vorigen Ansprüche 8 bis 10, **dadurch gekennzeichnet, dass** die Messeinrichtung zusätzlich wenigstens einen Temperatursensor (T1, T2) aufweist, der die Temperatur, z.B. die Innentemperatur, eines zu behandelnden und/oder zu untersuchenden Behälters messen kann.

## Claims

1. Method for testing and inspecting containers coated by means of a plasma treatment, e.g. plastic bottles, which are coated for instance with amorphous silicon oxide or carbon compounds, the method comprising that the containers are tested inline, i.e. at the speed at which they run through the production process and treatment process, by a measuring device trace-gas-analytically, e.g. mass-spectrometrically, for undesired foreign substances, such as acetaldehyde and/or antimony, escaping from the container material after a plasma coating treatment.

2. Method according to claim 1, the method comprising that the containers are tested inline within a test period of less than 0.1, 1, 10 or 100 ms per container.

3. Method according to one of the preceding claims, the method comprising that the air contained in the container is tested for undesired foreign substances escaping from the container material by means of a sampler.

4. Method according to one of the preceding claims, the method comprising that a standard gas, such as ambient air, technically purified air, an inert gas, a noble gas, a noble gas mixture or a combination of the aforementioned gases is blown into a container to be tested and that at least a part of the test gas escaping from the container is tested by a measuring device for undesired foreign substances escaping from the container material.

5. Method according to one of the preceding claims, the method comprising that if it is detected that a predefined limit value in the concentration is exceeded, for instance if the limit value is exceeded by more than 0.1, 1, 5, 10 or 100 % with respect to a specific undesired foreign substance, the tested container can be classified and/or marked, for instance, as inadequately coated and/or can be finally removed from the production and/or transferred to a new plasma coating treatment.

6. Method according to claim 5, the method comprising that the predefined limit value in the concentration with respect to a specific undesired foreign substance depends on the temperature of the container to be tested, for instance the internal temperature of the container to be tested, wherein for instance the tolerable predefined limit values in the concentration with respect to a specific undesired foreign substance for a container having an internal temperature of 30, 50°C or more are higher than the predefined limit values for a container having an internal temperature that corresponds to the room temperature.

7. Method according to one of the preceding claims, the method comprising that the container to be tested is inoculated with a tracer substance / with tracer substances, e.g. a food contact uncritical substance such as xenon, prior to the plasma treatment and that the container is then, after the plasma coating treatment, tested in a trace gas analysis for tracer substances escaping from the container material.

8. Apparatus for coating containers, e.g. plastic bottles, by means of a plasma treatment and for the testing and inspection of the container coatings obtained by the plasma treatment, comprising at least one plasma treatment unit for coating containers, and comprising at least one measuring device implemented as a mass spectrometer, **characterized in that** the measuring device (M) is configured to be capable of inline testing the containers coated by the plasma treatment unit (P) for undesired foreign substances escaping from the container material, i.e. at the speed at which they run through the production process and treatment process.

9. Apparatus according to claim 8, **characterized in that** the measuring device additionally comprises at least one sampler, configured such that the at least one sampler (N) can sample at least a part of the air contained in the container to be tested and pass it on to the measuring device (M).

10. Apparatus according to one of the preceding claims 8 to 9, **characterized in that** the measuring device additionally comprises at least one injection unit and at least one sampler, configured such that the at least one injection unit (I) can blow a standard gas into a container (B) to be tested and the at least one sampler (N) can sample at least a part of the test gas (K) escaping from a container to be tested and pass it on to the measuring device (M).

11. Apparatus according to one of the preceding claims 8 to 10, **characterized in that** the measuring device additionally comprises at least one temperature sensor (T1, T2) which can measure the temperature, for instance, the internal temperature of a container (B) to be treated and/or tested.

## Revendications

1. Procédé d'examen et de contrôle de récipients revêtus au moyen d'un traitement au plasma, tels que des bouteilles en plastique qui sont revêtues, par exemple, d'oxyde de silicium amorphe ou de composés de carbone, qui comprend l'examen des récipients après un traitement de revêtement au plasma, par analyse de gaz traces, par exemple par spectrométrie de masse, par un dispositif de mesure en ligne, c'est-à-dire à la vitesse à laquelle ils passent par le processus de production ou de traitement, à la recherche de substances étrangères indésirables, telles que l'acétaldéhyde et/ou l'antimoine, qui s'échappent du matériau de récipient.

2. Procédé selon la revendication 1, qui comprend l'examen en ligne des récipients à la recherche de substances étrangères indésirables dans un temps d'examen par récipient inférieur à 0,1, 1, 10 ou 100 ms.

3. Procédé selon l'une quelconque des revendications précédentes, qui comprend l'analyse, au moyen d'un renifleur, de l'air contenu dans le récipient à la recherche d'impuretés indésirables s'échappant du matériau de récipient.

4. Procédé selon l'une quelconque des revendications précédentes, qui comprend l'injection d'un gaz standard, comme par exemple de l'air ambiant, de l'air purifié techniquement, un gaz inerte neutre, un gaz noble, un mélange de gaz noble ou une combinaison desdits gaz, dans un récipient à examiner et au moins une partie du gaz échantillon sortant du récipient est inspectée par un dispositif de mesure à la recherche de substances étrangères indésirables sortant du matériau de récipient.

5. Procédé selon l'une quelconque des revendications précédentes, dans lequel lors de la détection du dépassement d'une valeur limite prédéfinie dans la concentration, par exemple en cas de dépassement de la valeur limite de plus de 0,1, 1, 5, 10 ou 100 %, par rapport à une substance étrangère indésirable particulière, le récipient examiné, par exemple, est classé comme insuffisamment revêtu et/ou peut être marqué, et/ou est définitivement évacué de la production, et/ou est soumis à un nouveau traitement de revêtement au plasma,

6. Procédé selon la revendication 5, dans lequel la valeur limite prédéfinie dans la concentration par rapport à une substance étrangère indésirable particulière dépend de la température du récipient à examiner, par exemple de la température interne du récipient à examiner, dans lequel, par exemple, la valeur limite prédéfinie tolérable dans la concentration par rapport à une substance étrangère indésirable particulière pour un récipient avec une température interne de 30, 50°C ou plus, est supérieure aux valeurs limites prédéfinies pour un récipient avec une température interne qui est égale à la température ambiante.

7. Procédé selon l'une quelconque des revendications précédentes, qui comprend l'inoculation au récipient à examiner d'une substance traceuse/de substances traceuses, par exemple une substance non critique d'un point de vue alimentaire, telle que le xénon, avant le traitement de revêtement au plasma, et l'examen des gaz traces dans le récipient après le traitement de revêtement au plasma concernant des substances traceuses sortant du matériau de récipient.

8. Dispositif pour revêtir des récipients, par exemple de bouteilles en plastique, au moyen d'un traitement au plasma, ainsi que pour l'examen et le contrôle des revêtements de récipients effectués par le traitement au plasma, avec au moins une unité de traitement au plasma pour le revêtement de récipients, et avec au moins un dispositif de mesure conçu sous la forme d'un spectromètre de masse, **caractérisé en ce que** le dispositif de mesure (M) est configuré de manière à pouvoir examiner en ligne les récipients revêtus par l'unité de traitement au plasma (P) à la recherche de substances étrangères indésirables sortant du matériau de récipient, c'est-à-dire à la vitesse à laquelle les récipients passent par le processus de production ou de traitement.

9. Dispositif selon la revendication 8, **caractérisé en ce que** le dispositif de mesure présente en outre au moins un renifleur, qui est configuré de telle sorte que le au moins un renifleur (N) puisse recevoir au moins une partie de l'air se trouvant dans le récipient à examiner et le transmettre au dispositif de mesure (M).

10. Dispositif selon l'une quelconque des revendications 8 à 9, **caractérisé en ce que** le dispositif de mesure présente en outre au moins une unité d'injection et au moins un renifleur, configurés de manière à ce que la au moins une unité d'injection (I) puisse injecter un gaz standard dans un récipient (B) à analyser, et le au moins un renifleur (N) puisse recevoir au moins une partie du gaz échantillon (K) sortant d'un récipient à analyser et le transmettre au dispositif de mesure (M).

11. Dispositif selon l'une quelconque des revendications précédentes 8 à 10, **caractérisé en ce que** le dispositif de mesure présente en outre au moins un capteur de température (T1, T2) qui peut mesurer la température, par exemple la température intérieure, d'un récipient à traiter et/ou à examiner.
